# EUROPÄISCHE PATENTSCHRIFT

(11) **EP 3 668 375 B1**
(45) Veröffentlichungstag und Bekanntmachung des Hinweises auf die Patenterteilung: **11.03.2026**
(21) Anmeldenummer: 18758842.1
(22) Anmeldetag: 14.08.2018
(51) Int. Cl.: A61B 5/00, A61B 5/103, A45D 44/00

(54) **SYSTEM UND VERFAHREN ZUM ERMITTELN EINES HAARZUSTANDS**
SYSTEM AND METHOD FOR DETERMINING THE CONDITION OF HAIR
SYSTÈME ET PROCÉDÉ POUR DÉTERMINER UN ÉTAT DES CHEVEUX

(30) Priorität: 16.08.2017 DE 102017214250
(43) Veröffentlichungstag der Anmeldung: 24.06.2020
(73) Patentinhaber: Henkel AG & Co. KGaA, 40589 Düsseldorf (DE)
(72) Erfinder: MATHIASZYK, Carsten, 45277 Essen (DE); LECHNER, Torsten, 40764 Langenfeld (DE)
(86) Internationale Anmeldenummer: PCT/EP2018/071984
(87) Internationale Veröffentlichungsnummer: WO 2019/034635

(56) Entgegenhaltungen:
- WO-A2-2010/076104
- US-A1- 2015 045 631
- US-A1- 2015 342 515
- US-A1- 2017 038 297

## Beschreibung

Die Erfindung betrifft ein System und Verfahren zum Ermitteln eines Haarzustands und Verfahren zum Ermitteln einer Empfehlung bezüglich Haaren eines Nutzers.

In vielen Bereichen des täglichen Lebens gibt es seit einiger Zeit einen Trend zu personalisierten Programmen, die auf individuelle Voraussetzungen und Bedürfnisse gezielt eingehen können, beispielsweise in einem Ernährungs- oder Gesundheitsbereich, aber auch in einem Bereich personalisierter Kosmetik. Diese kann es einem Nutzer ermöglichen, gezielt Kosmetikprodukte zu finden und/oder Pflegehinweise zu erhalten, die auf individuelle Bedürfnisse seiner Haare abgestimmt sind, und somit eine besonders hohe Wirksamkeit ermöglichen.

Bei einer Behandlung von Haar mit kosmetischen Produkten kann eine Wirkung des Produkts, z.B. eine Intensität einer Färbung, eine Wirksamkeit eines Pflegeprodukts oder eine Haarumformungswirkung einer Dauerwelle, stark von einem Schädigungsgrad des Haars abhängen.

Deshalb kann eine Ermittlung einer Schädigung des Haars von großer Bedeutung sein.

Die Schädigung des Haars kann durch natürliche oder künstlich herbeigeführte Vorgänge passieren. Der wichtigste Schädigungstyp kann dabei eine oxidative Schädigung sein.

Die natürlichen Vorgänge können beispielsweise eine kombinierte (z.B. gleichzeitige) Einwirkung von UV-Licht und Sauerstoff (O₂) auf das Haar aufweisen.

Die künstlich herbeigeführten Vorgänge können dabei beispielsweise ein Anwenden von Haarfärbemitteln (auch als Colorationen bezeichnet, wozu hierin auch ein Blondieren gezählt wird), und/oder ein Stylen oder Umformen des Haars (z.B. ein Erzeugen einer Dauerwelle) aufweisen.

Dabei kann neben erwünschten kosmetischen Effekten, wie z.B. einer Aufhellung des Haars, auch eine starke Schädigung des Haars auftreten, beispielsweise bei einer Verwendung von Oxidationsmitteln.

Beim geschädigten Haar kann beispielsweise ein Cysteinsäuregehalt erhöht sein wegen einer Oxidation der im Haar sehr häufig vorkommenden Aminosäuren Cystin und Cystein zu Cysteinsäure.

Die Oxidation des Cystins/Cysteins zu Cysteinsäure kann die mechanische Stabilität des Haars zerstören und bei mehrfachen Anwendungen sogar zu einem vollständigen Haarbruch führen. Allerdings können bereits vorher makroskopisch wahrnehmbare, z.B. fühlbare, Eigenschaften des Haars, beispielsweise eine Oberflächenbeschaffenheit, z.B. eine Oberflächenrauhigkeit, negativ beeinflusst werden. Geschädigtes Haar kann beispielsweise eine höhere Oberflächenrauhigkeit aufweisen als ungeschädigtes Haar.

Ergebnisse kosmetischer Behandlungen können von weiteren Eigenschaften des behandelten Haars abhängen, beispielsweise (insbesondere bei einer Coloration) von einer Haarfarbe, von einer Haarstruktur (insbesondere bei einem Styling, z.B. einer Dauerwelle, einer Glättung usw.), von einem Feuchtigkeitsgehalt (bei einem Pflegeprodukt), usw.

Einem Nutzer können Vorrichtungen (Werkzeuge, Tools), zum Beispiel Bürsten, Kämme, handliche Nahinfrarot- (NIR-)Messgeräte, Mikroskope oder weitere/andere Vorrichtungen, bereitgestellt sein, mittels welcher, beispielsweise unter Verwendung eines so genannten "Smart Device" eine Haarschädigung oder ein anderer Haarzustandsparameter ermittelt werden kann. Unter den Begriff "Smart Device" fallen elektronische Geräte, die in der Regel mit anderen Geräten oder Netzwerken über verschiedene drahtlose Protokolle wie Bluetooth, NFC, Wi-Fi, 3G usw. verbunden sind und eine sensorgestützte, elektronische Datenverarbeitung aufweisen. Beispiele für "Smart Devices" sind Smartphones, Phablets, Tablets, Smart Bands, Smart Mirrors, Smart Watches oder Smart Key Chains. Ein Ergebnis einer Haarschädigungsuntersuchung kann beispielsweise auf einem Smartphone, einem Tablet oder ähnlichem dargestellt werden. Allerdings kann es unmöglich sein, mehrere unterschiedliche Ergebnisse zusammenzufassen und, beispielsweise gemeinsam, auszuwerten.

US 2015/0342515 A1 beschreibt ein Hairstyling-Werkzeug vom Kamm- oder Bürstentyp, umfassend einen Griff und einen Körper, eine elektrische Batterie, einen Kraftsensor, eine elektronische Einheit, welche dazu eingerichtet ist, die von den Sensoren bereitgestellten Signale zu erfassen und zu verarbeiten, ein drahtloses Kommunikationsmittel zur Übertragung von Daten an eine entfernte Einheit, mittels dessen Informationen über die vom Werkzeug ausgeführten Bewegungen und die auf es einwirkenden Kräfte an die entfernte Einheit übermittelt werden können, wobei die entfernte Einheit dem Benutzer des Werkzeugs Informationen über das zu stylende Haar zurücksenden kann.

US 2015/0045631 A1 beschreibt ein integriertes System für Haut- und Haarpflege, welches eine personalisierte Analyse der spezifischen Situation eines Benutzers bietet und dem Benutzer Empfehlungen für Verbraucherprodukte zur Verfügung stellt. Das System ist so implementiert, dass der Benutzer über ein mobiles Gerät auf sein persönliches Konto zugreifen kann. Das Gerät ist ein tragbares Instrumentenpaket, das Haut- und Umweltdaten misst und überträgt. Das System integriert ein oder mehrere solcher Geräte mit einem Kommunikationsnetzwerk und einem entfernten Datenprozessor, um die Hautgesundheit und die allgemeine Gesundheit einer Person zu verbessern.

US 2017/0038297 A1 beschreibt eine Vorrichtung und ein Verfahren zur individuellen Haarfärbung. Das Verfahren umfasst folgende Schritte: Durchführung einer Vielzahl von Lichtstreumessungen an einer Haarprobe, wobei die Haarprobe für jede Lichtstreumessung aus einer jeweils unterschiedlichen Richtung beleuchtet wird; Vergleich der Ergebnisse der Lichtstreumessungen; Berechnung eines anfänglichen Schadenszustands des Haares der Probe anhand der Ergebnisse des Vergleichs der Lichtstreumessungen; Ermittlung eines anfänglichen Farbzustands des Haares der Probe; sowie Berechnung einer Haarfärbezusammensetzung, die voraussichtlich die Haarprobe vom anfänglichen Farbzustand in einen Ziel-Farbzustand überführt.

WO 2010/076104 A2 beschreibt Mittel zur schonenden oxidativen Färbung keratinischer Fasern, insbesondere menschlicher Haare, die durch Vermischen einer Färbezubereitung, enthaltend Oxidationsfarbstoffvorprodukte, und einer Oxidationsmittelzubereitung herstellt werden, wobei sich die Oxidationsmittelzubereitung dadurch auszeichnet, dass sie als Oxidationsmittel kein Wasserstoffperoxid, sondern Natriumchlorit enthält.

Gemäß der Erfindung werden ein System zum Ermitteln eines Haarzustands gemäß Patentanspruch 1 und ein Verfahren zum Ermitteln eines Haarzustands gemäß Patentanspruch 6 bereitgestellt. Weitere Aspekte sind in den Patentansprüchen 2 bis 5 und 7 bis 10 dargelegt.

In verschiedenen Ausführungsbeispielen wird eine Vorrichtung (zum Beispiel ein Smartphone, Tablet, Smart Mirror oder Ähnliches) bereitgestellt, welche geeignet sein kann, sich mit allen bekannten oder zukünftigen Vorrichtungen/Werkzeugen (zum Beispiel zu einem Ermitteln eines Haarzustandsparameters), vorzugsweise drahtlos, zu verbinden (zum Beispiel mittels Bluetooth) und somit eine Haarqualität (auch als Haarzustand bezeichnet) zu ermitteln.

Als Vorrichtungen/Werkzeuge können dabei beispielsweise Bürsten, Kämme, Mikroskope und oder Nahinfrarot-Geräte zum Einsatz kommen.

In verschiedenen Ausführungsbeispielen können die einzelnen Untersuchungsergebnisse mittels einer Software (beispielsweise einer App) analysiert werden, beispielsweise einzelnd ausgewertet werden. In verschiedenen Ausführungsbeispielen kann mittels der Vorrichtung ein Vergleichen unterschiedlicher Ergebnisse erfolgen und ein gemeinsamer Haarzustandswert ermittelt werden, sodass ein verbesserter Hinweis auf den Haarzustand bereitgestellt werden kann.

Auf Grundlage der erzeugten Daten kann die Software dem Nutzer geeignete Haarbehandlungsprodukte und/oder Haarbehandlungsverfahren empfehlen. Außerdem kann es möglich sein, die empfohlenen Haarbehandlungsprodukte direkt mittels der Software online zu bestellen und/oder es kann angegeben, beispielsweise mittels einer Anzeige auf einem Bildschirm und/oder einer Ansage mittels eines Lautsprechers, werden, wo diese Haarbehandlungsprodukte erhältlich sind.

In verschiedenen Ausführungsbeispielen können einem Nutzer passend zu seinem Haarzustand Haarbehandlungsproduktempfehlungen bezüglich Haarfärbemitteln (Haarcolorationen), welche auch Mittel zum Blondieren einschließen können, Dauerwelle, Haarpflege und/oder Haarstyling angeboten werden.

Außerdem kann es einem Nutzer ermöglicht werden, seinen Haarzustand noch genauer zu ermitteln.

Im Rahmen der vorliegenden Anmeldung wird ein System zum Ermitteln eines Haarzustands bereitgestellt. Das System weist eine tragbare Sensorvorrichtung mit mindestens einem Sensor zum Erfassen mindestens eines Sensorwerts an Haaren eines Nutzers und eine Datenverarbeitungsvorrichtung auf.

Die Datenverarbeitungsvorrichtung ist eingerichtet, den Haarzustand des Nutzers basierend auf dem erfassten mindestens einen Sensorwert zu ermitteln. Allerdings kann die Sensorvorrichtung gemäß verschiedenen Ausführungsbeispielen modular aufgebaut sein.

Das die eine Sensorvorrichtung und die Datenverarbeitungsvorrichtung aufweisende System bildet eine Basisausführung, welche mindestens eine weitere Sensorvorrichtung aufweist. Dabei ist die Datenverarbeitungsvorrichtung bereits bei der Basisausführung eingerichtet zu ermitteln, ob ihr lediglich Daten (Sensorwerte) von der Sensorvorrichtung bereitgestellt werden, oder ob ihr ferner mindestens ein weiterer Sensorwert von einer weiteren Sensorvorrichtung bereitgestellt wird.

Abhängig von dem Ergebnis des Ermittelns wird mittels der Datenverarbeitungsvorrichtung der Haarzustand basierend auf dem von der Sensorvorrichtung bereitgestellten mindestens einen Sensorwert ermittelt, oder basierend auf dem von der Sensorvorrichtung bereitgestellten mindestens einen Sensorwert in Kombination mit dem von der weiteren Sensorvorrichtung bereitgestellten mindestens einen weiteren Sensorwert ermittelt.

In verschiedenen Ausführungsbeispielen kann das System zum Ermitteln des Haarzustands eine Eingabevorrichtung aufweisen.

In verschiedenen Ausführungsbeispielen kann die Eingabevorrichtung genutzt werden, um ein Wunschergebnis einzugeben, beispielsweise eine Wunschhaarfarbe, einen Wunsch-Pflegezustand, ein gewünschtes Styling (z.B. Locken mit einer abgestuften Lockigkeit oder ähnliches).

Zusätzlich kann die Eingabevorrichtung genutzt werden, um neben Sensor-ermittelten Haarzustandsparametern empirisch ermittelte Haarzustandsparameter einzugeben, beispielsweise Ergrauungsgrad, Haardicke oder Haarkräuselungsgrad, welche beispielsweise durch Betrachten oder Anfassen der Haare bestimmt werden.

In verschiedenen Ausführungsbeispielen kann die Anzeige- und Eingabevorrichtung mit der Sensorvorrichtung eine integrierte Einheit bilden, beispielsweise beim Verwenden einer Kamera und/oder eines Mikrofons eines Smartphones als Sensor, oder beispielsweise indem ein Nahinfrarotspektrometer mit einer Datenverarbeitungsvorrichtung und einem z.B. berührungsempfindlichen Display ausgerüstet ist.

In verschiedenen Ausführungsbeispielen kann das System zum Ermitteln des Haarzustands eine Ausgabevorrichtung, z.B. eine Anzeigevorrichtung aufweisen.

In verschiedenen Ausführungsbeispielen kann mindestens eine von der Sensorvorrichtung, der weiteren Sensorvorrichtung und der Datenverarbeitungsvorrichtung zusätzlich zur Anzeigevorrichtung oder alternativ dazu eine akustische Ausgabevorrichtung aufweisen, z.B. einen Lautsprecher.

In verschiedenen Ausführungsbeispielen kann mindestens eine von der Sensorvorrichtung, der weiteren Sensorvorrichtung und der Datenverarbeitungsvorrichtung zusätzlich zum berührungsempfindlichen Bildschirm als Eingabevorrichtung oder alternativ dazu eine akustische Eingabevorrichtung aufweisen, z.B. ein Mikrofon.

In einem Fall, dass die Sensorvorrichtung oder die weitere Sensorvorrichtung das Mikrofon aufweist, kann das Mikrofon in verschiedenen Ausführungsbeispielen auch als Eingabemikrofon genutzt werden.

In verschiedenen Ausführungsbeispielen kann als Eingabevorrichtung alternativ oder zusätzlich eine andere herkömmliche Eingabevorrichtung bereitgestellt sein, z.B. eine Tastatur, eine Maus, etc.

In verschiedenen Ausführungsbeispielen kann mittels des Systems, z.B. mittels der Sensorvorrichtung ( ergänzt durch die weitere Sensorvorrichtung) in Verbindung mit der Datenverarbeitungsvorrichtung, eine standardisierte und objektive Beurteilung des Behandlungsergebnisses ermöglicht sein. Zu dem Zweck kann mittels des mindestens einen Sensors der Sensorvorrichtung ( zusätzlich durch den mindestens einen weiteren Sensor der weiteren Sensorvorrichtung) der Haarzustand des Nutzers nach der Behandlung mit dem Haarbehandlungsmittel ermittelt werden.

Das Ermitteln des Haarzustands umfasst ein Ermitteln eines Schädigungsgrads des

Haars, kann aber außerdem auch ein Ermitteln einer Haarfarbe und/oder ein Ermitteln einer Haarfeuchtigkeit umfassen. Bei dem Schädigungsgrad kann beispielsweise einen oxidativen Schädigungsgrad umfassen.

In verschiedenen Ausführungsbeispielen ist das System zum Ermitteln des Haarzustands so eingerichtet, dass mittels der Sensorvorrichtung und/oder der weiteren Sensorvorrichtung ein Ermitteln eines Grads der oxidativen Haarschädigung durch ein Ermitteln eines Gehaltes an Cysteinsäure exakt ermittelbar ist. Der Sensor kann dabei mindestens ein optischer Sensor sein, welcher eingerichtet sein kann, eine oder mehrere Aufnahmen in einem Fluoreszenzbereich und/oder in einem Infrarotbereich (IR-Bereich), vorzugsweise in einem Nahinfrarot-Bereich (NIR-Bereich), zu machen.

Der Fluoreszenzbereich kann in verschiedenen Ausführungsbeispielen ein Wellenlängenbereich sein, in welchem geschädigtes Haar Eigenfluoreszenz emittiert und/oder ein Wellenlängenbereich, in welchem Fluoreszenzfarbstoffe, welche von geschädigtem Haar stärker adsorbiert werden als von ungeschädigtem Haar, Fluoreszenzlicht emittieren.

Der IR-Bereich, insbesondere der NIR-Bereich, kann in verschiedenen Ausführungsbeispielen ein Wellenlängenbereich sein, in welchem geschädigtes Haar Absorptionsstrukturen aufweist, z.B. in welchem Cysteinsäure Licht absorbiert.

Ungeschädigtes Haar kann typischerweise einen Cysteinsäuregehalt in einem Bereich von etwa 0.5% bis etwa 1% (nach Gewicht) aufweisen. Bei Vorliegen einer Schädigung, beispielsweise infolge mehrfachen Ultrablondierens und/oder anderer Schädigungsmechanismen, kann der Cysteinsäuregehalt auf über 15% (Gew.) ansteigen.

Diese Eigenschaft wird genutzt, um den Schädigungsgrad des Haars als einen Gehalt an Cysteinsäure zu quantifizieren.

In verschiedenen Ausführungsbeispielen kann geschädigtes Haar eine Eigenfluoreszenz zeigen, welche genutzt wird, um mittels Erfassens einer Fluoreszenzintensität des Haars den Schädigungsgrad zu ermitteln.

In verschiedenen Ausführungsbeispielen kann das Haar mit einer Fluoreszenzfarbstofflösung benetzt werden, welche von geschädigtem Haar besser adsorbiert wird als von ungeschädigtem Haar, wobei die Fluoreszenzfarbstofflösung Rhodamin B, Cumarin und/oder Fluoreszein aufweisen kann.

In verschiedenen Ausführungsbeispielen kann zum Ermitteln der Fluoreszenzintensität des Haars das Haar mit UV-Licht (z.B. mit Licht in einem Wellenlängenbereich von etwa 315 nm bis etwa 380 nm) belichtet werden. Zu dem Zweck kann die Sensorvorrichtung oder die weitere Sensorvorrichtung mit einer UV-Lichtquelle versehen sein. Die UV-Lichtquelle kann eine UV-LED oder eine andere geeignete Lichtquelle sein, z.B. eine herkömmliche UV-Lampe, wie sie bei einer Echtheitsprüfung von Geldscheinen verwendet wird.

Während des Belichtens kann Fluoreszenzlicht registriert werden, welches von dem Haar emittiert wird. Die Fluoreszenzintensität kann anhand des registrierten Lichts ermittelt werden. Unter Einbeziehung der Fluoreszenzintensität des Haars kann dann der Schädigungsgrad des Haars ermittelt werden.

Dementsprechend kann die Sensorvorrichtung oder die weitere Sensorvorrichtung in verschiedenen Ausführungsbeispielen einen optischen Sensor aufweisen, der zumindest im Fluoreszenz-Wellenlängenbereich empfänglich ist, beispielsweise eine Kamera, ein Photometer, ein Colorimeter und/oder ein Spektrometer. In verschiedenen Ausführungsbeispielen kann zwischen dem Haar und dem optischen Sensor in verschiedenen Ausführungsbeispielen ein Filter angeordnet sein.

In verschiedenen Ausführungsbeispielen kann ein Infrarot-(IR-)Spektrum, insbesondere ein Nahinfrarot- (NIR-)Spektrum gewonnen werden, beispielsweise mittels ATR-(Nah-)Infrarotspektroskopie (von Englisch "attenuated total reflection", auf Deutsch "abgeschwächte Totalreflexion"). Durch eine Anwendung von mathematischen Modellen kann mittels Vermessung von Kalibrier-Haarproben, welche einen anhand eines bekannten analytischen Verfahrens ermittelten Cysteinsäuregehalt aufweisen, ein mathematisches Modell erstellt werden.

Bei einer Analyse eines am Haar des Nutzers aufgenommenen IR-Spektrums, insbesondere NIR-Spektrums, oder zumindest eines Teils davon, kann in verschiedenen Ausführungsbeispielen das Modell eine Berechnung des Gehalts an Cysteinsäure, und damit der Haarschädigung, erlauben. Eine Analyse von zumindest einem Teil des Spektrums und eine Anwendung des Modells kann dabei mittels der Datenverarbeitungsvorrichtung, beispielsweise (mit geeigneten Apps) mittels bekannter Smartphones, Tablets o.ä., ausgeführt werden.

Die Sensorvorrichtung oder die weitere Sensorvorrichtung kann eine IR-Lichtquelle, insbesondere NIR-Lichtquelle, zum Belichten des Haars mit IR-Licht, insbesondere NIR-Licht, aufweisen. In der vorliegenden Beschreibung wird für Licht mit einer Wellenzahl in einem Bereich von 12.820 bis 4000 cm⁻¹ der Begriff Nahinfrarot (NIR) verwendet, und für Licht mit einer Wellenzahl in einem Bereich von 3999 bis 400 cm⁻¹ der Begriff Infrarot (IR).

Das Ermitteln des Schädigungsgrads von Haar kann gemäß verschiedenen Ausführungsbeispielen entweder unter Nutzung des Nahinfrarot-Bereichs ausgeführt werden, d.h. mittels Bestrahlens des Haars mit dem Nahinfrarotlicht und Spektralanalyse von zumindest einem Teil des NIR-Lichts, nachdem dieses mit dem Haar gewechselwirkt hat, oder unter Nutzung des Infrarot-Bereichs, d.h. mittels Bestrahlens des Haars mit Infrarotlicht und Spektralanalyse von zumindest einem Teil des IR-Lichts, nachdem dieses mit dem Haar gewechselwirkt hat, oder unter Verwendung von sowohl dem Nahinfrarot- als auch dem (restlichen) Infrarotbereich, d.h. mittels Bestrahlens des Haars mit Nahinfrarot- und Infrarotlicht und Spektralanalyse von zumindest einem Teil des NIR- und zumindest einem Teil des IR-Lichts, nachdem dieses mit dem Haar gewechselwirkt hat.

In verschiedenen Ausführungsbeispielen kann ein vermessener Nahinfrarot (NIR)-Bereich Wellenzahlen von etwa 12820 cm⁻¹ bis etwa 4000 cm⁻¹, z.B. von etwa 5022 cm⁻¹ bis etwa 4020 cm⁻¹, aufweisen. Dieser Wellenlängenbereich kann u.a. charakteristische Oberton- und Kombinationsschwingungen von z.B. CH-, OH- und NH-Gruppen aufweisen.

In verschiedenen Ausführungsbeispielen kann der zumindest eine Teil des Nahinfrarot- und/oder Infrarotlichts einen (Infrarot-)Wellenzahlbereich von etwa 1100 cm⁻¹ bis etwa 1000 cm⁻¹, z.B. um etwa 1040 cm⁻¹, aufweisen. Hier können sich u.a. die relevanten Absorptionsbanden der zu analysierenden Komponente Cysteinsäure befinden.

In verschiedenen Ausführungsbeispielen kann anhand von Ergebnissen einer quantitativen rechnergestützten Auswertung (auch als chemometrischen Analyse bezeichnet) für eine Mehrzahl von Kalibrationshaarproben in Kombination mit mittels eines unabhängigen Verfahrens, z.B. mittels Hochdruckflüssigchromatographie, für dieselben Kalibrationshaarproben gewonnenen Werten für einen Cysteinsäuregehalt der jeweiligen Kalibrationshaarprobe, ein Kalibrationsmodell erstellt werden.

Liegt das Kalibrationsmodell vor, kann in verschiedenen Ausführungsbeispielen sehr einfach für das zu messende Haar anhand des für das aufgenommene (N)IR-Spektrum die Konzentration der Cysteinsäure (als Maß für die Haarschädigung) aus den Spektren im Vergleich mit den Kalibrationsspektren berechnet werden.

Neben der direkten Bestimmung des Cysteinsäuregehalts über die charakteristischen Absorptionsbanden der Cysteinsäure, insbesondere im Bereich von 5022 cm⁻¹ bis 4020 cm⁻¹, kann auch eine indirekte Bestimmung des Cysteinsäuregehalts erfolgen.

Zwischen dem Gehalt an Cysteinsäure und dem Gehalt an Melanin besteht eine inverse Korrelation, die indirekt wieder - über die Bestimmung des Melaningehaltes - die Bestimmung des Gehalts an Cysteinsäure ermöglicht. Der Prozess der oxidativen Schädigung (Bildung von Cysteinsäure) beim Blondieren oder Färben von Haaren ist mit dem Abbau von Melanin verknüpft.

Mit Hilfe von kurzwelliger Nahinfrarotspektroskopie in einem Wellenzahlbereich von 12.820 bis 7692 cm⁻¹, einem Wellenzahlbereich in dem Cysteinsäure keine charakteristische Absorption zeigt, lassen sich zuverlässige Kalibriermodelle erstellen, die eine Korrelation zwischen dem kurzwelligen Nahinfrarotspektrum und dem Cysteinsäuregehalt herstellen.

In verschiedenen Ausführungsbeispielen kann das Ermitteln des Schädigungsgrads des Haars ein Ermitteln einer Schädigung des Haars mittels Interferenzreflexionsmikroskopie aufweisen.

Mit Hilfe der Interferenzreflexionsmikroskopie können sehr dünne Haarstrukturen untersucht werden. Die Interferenzreflexionsmikroskopie beruht auf der Bildung von Interferenzen, die entstehen, wenn Licht an der oberen und der unteren Grenzfläche einer Struktur reflektiert wird und reflektiertes Licht von beiden Grenzflächen miteinander interferiert. Dadurch entstehen Interferenzmuster, die beobachtet werden können, und die Aufschluss über die Dicke der Struktur liefern. Die entstehenden Interferenzfarben lassen Strukturmessungen im Bereich unter 200 nm zu. Durch Betrachten der Interferenzfarben durch ein Lichtmikroskop können diese Strukturmessungen entsprechend zu mikroskopisch erkennbaren Strukturen zugeordnet werden.

Gemäß verschiedenen Ausführungsformen kann dies auf die Cuticula von Haaren angewendet werden, um den Schädigungsgrad der Haare zu ermitteln.

Dabei kann während eines Belichtens des Haars mit Licht von dem Haar abgestrahltes Licht registriert werden. Basierend auf dem registrierten Licht können erste Bereiche des Haars ermittelt werden, die das Licht mit höherer Interferenz reflektieren und deshalb in einer Aufnahme des Lichts heller erscheinen, und zweite Bereiche des Haars, die das Licht mit niedrigerer Interferenz reflektieren und deshalb dunkler erscheinen. Basierend auf den Größen der ersten Bereiche und der zweiten Bereiche kann der Schädigungsgrad des Haars ermittelt werden.

Das Haar weist eine Cuticula, einen Kortex und Mark auf.

Wird das Haar mit Licht (z.B. Weißlicht) bestrahlt, welches mittels einer Lichtquelle der Sensorvorrichtung oder der weiteren Sensorvorrichtung bereitgestellt werden kann, z.B. einer Weißlicht-LED, so wird ein Teil des Lichts an der Außenfläche der Cuticula reflektiert und ein Teil wird an der Grenzfläche zwischen Cuticula und Kortex reflektiert (insbesondere dann, wenn die Cuticula sich von dem Kortex abgehoben oder abgelöst hat, was typischerweise einer Schädigung des Haares entspricht). Die reflektierten Teile interferieren und bilden ein Interferenzmuster.

Die Sensorvorrichtung oder die weitere Sensorvorrichtung kann eine Kamera aufweisen, die an ein Interferenzreflexionsmikroskop der Sensorvorrichtung oder der weiteren Sensorvorrichtung gekoppelt sein kann. Das Interferenzreflexionsmikroskop, welches einen Vergrößerungsfaktor in einem Bereich von etwa 10-1000 aufweisen kann, z.B. etwa 200-400, kann auf das Haar gerichtet sein und das vom Haar, z.B. von einer oder mehreren Haarfasern, reflektierte Licht auf einen Detektor der Kamera abbilden, welcher das reflektierte Licht als mindestens ein (digitales) Foto registrieren kann.

Die Datenverarbeitungsvorrichtung kann in verschiedenen Ausführungsbeispielen für das oder jedes der Fotos Art und/oder Anzahl der Interferenz-Muster der Haare mittels einer Bildanalyse-Software ermitteln, sie mit einem auf gleiche Art erstellten Kalibriermodell vergleichen und so einen Schädigungsgrad der Haare ermitteln. Bei mehreren Fotos kann die Datenverarbeitungsvorrichtung beispielsweise einen Mittelwert der für die Fotos ermittelten Schädigungsgrade bilden.

Das Interferenzreflexionsmikroskop, die Kamera, die Datenverarbeitungsvorrichtung und gegebenenfalls auch die Lichtquelle können in verschiedenen Ausführungsbeispielen durch ein Smartphone realisiert werden, das mit einem Mikroskop-Objektiv für Smartphones ausgestattet ist, das auch für Interferenzreflexionsmikroskopie geeignet ist. Um stabile Untersuchungsbedingungen zu gewährleisten, können die Haare in verschiedenen Ausführungsbeispielen auf einem Träger angeordnet sein oder werden. Dieser kann beispielsweise mittels der Datenverarbeitungsvorrichtung (z.B. des Smartphones) oder eines Aufsatzes für die Datenverarbeitungsvorrichtung bereitgestellt werden.

Beispielsweise können mittels eines tragbaren elektronischen Geräts (wie beispielsweise eines Smartphones, eines Tablets, etc.) mit Mikroskop-Aufsatz (wie beispielsweise einem Scrona µpeek) in Kombination mit einem Interferenz-Schieber (engl. interference slider, wie beispielsweise angeboten von Hirox Ltd.) Haare bei 350fachen Vergrößerung aufgenommen werden.

Der Flächenteil der interferierenden Haarstrukturen, das heißt der Anteil der hellen (geschädigten) Bereiche, an dem Gesamtbereich des Haars in dem Foto, liegt beispielsweise zwischen 1% und 50%, z.B. zwischen 5% und 30%.

Die Datenverarbeitungseinrichtung kann dann von dem ermittelten Flächenanteil heller Flächen auf den Schädigungsgrad schließen, beispielsweise mit Hilfe einer Tabelle, die Bereiche von Flächenanteilen Schädigungsgraden zuordnet.

Eine Art der Schädigung, welche mittels der Interferenzreflexionsmikroskopie ermittelbar ist, kann beispielsweise eine mechanische Schädigung sein, wie sie z.B. durch Dehnung des Haars verursacht werden kann.

In verschiedenen Ausführungsbeispielen kann die äußere Haarschädigung, beispielsweise in Form der Oberflächenrauhigkeit, mittels der Sensorvorrichtung oder der weiteren Sensorvorrichtung, die einen Sensor zum Erfassen akustischer Emissionen, z.B. ein Mikrofon, aufweist, z.B. mittels eines Kontaktmikrofonkammes, ermittelt werden.

Hierin kann der Sensor zum Erfassen akustischer Emissionen der Einfachheit halber auch als Mikrofon bezeichnet werden. Sofern nicht anders angegeben und für die beschriebene Funktion geeignet, kann der Sensor zum Erfassen akustischer Emissionen allerdings auch beispielsweise ein Beschleunigungssensor (der geeignet sein kann, Beschleunigungen infolge akustischer Emissionen in einem gewissen Frequenzbereich zu erfassen) oder ähnliches sein.

Die Sensorvorrichtung oder die weitere Sensorvorrichtung kann in verschiedenen Ausführungsbeispielen als Kontaktmikrofonkamm ausgeführt sein. Der Kontaktmikrofonkamm kann in verschiedenen Ausführungsbeispielen einen im Wesentlichen handelsüblichen Haarkamm aufweisen, an dem ein oder mehrere von außen angebrachte Messsysteme für akustische Emissionen (d.h. Schallemissionen; als ein beispielhaftes Messsystem für akustische Emissionen kann ein Kontaktmikrofon der Fa. Korg genutzt werden) und/oder Messsonden für akustische Emissionen angeschlossen sein. Alternativ kann die Sensorvorrichtung oder die weitere Sensorvorrichtung als Kontaktmikrofonbürste ausgeführt sein.

In verschiedenen Ausführungsbeispielen können mittels der Sensorvorrichtung, die das Mikrofon aufweist, Signale von erzeugtem Schall oder Vibration aufgenommen werden, die bei einem Durchkämmen von Haaren eines Nutzers entstehen.

Die Sensorvorrichtung oder die weitere Sensorvorrichtung kann in verschiedenen Ausführungsbeispielen ein oder mehrere Messsysteme für akustische Emissionen und/oder Sonden aufweisen, z.B. eines oder mehrere Kontaktmikrofone und/oder einen Beschleunigungssensor. Die Sensorvorrichtung oder die weitere Sensorvorrichtung kann in verschiedenen Ausführungsbeispielen ferner einen internen oder externen Verstärker aufweisen zum Verstärken von mittels des Messsystems für akustische Emissionen gemessenen Signalen.

In verschiedenen Ausführungsbeispielen kann eine Analyse einer Haarschädigung bereitgestellt werden mittels Digitalisierens einer Information von einem Mikrofon (und mindestens eines weiteren Sensors) und Bereitstellens dieser Daten.

Die Information kann nach einem Verarbeiten bereitgestellt werden oder sein für ein Vergleichen mit bereits aufgezeichneten Beispielen (Referenzdaten). Für die Referenzdaten kann ein Haarschädigungsgrad bekannt sein, so dass als ein Ergebnis des Vergleichs beispielsweise der Haarschädigungsgrad der ähnlichsten Referenzdaten als Ergebnis in digitaler Form bereitgestellt, z.B. zurückübermittelt, werden kann.

In verschiedenen Ausführungsbeispielen können für die Quantifizierung des Cysteinsäuregehalts (z.B. mittels Fluoreszenzanalyse und/oder mittels (N)IR-Spektroskopie) oder des (äußeren) Schädigungsgrads (z.B. mittels Interferenzreflexionsmikroskopie und/oder mittels akustischer Analyse) geeignete mathematische Modelle der Prädiktiven Analytik genutzt werden.

In verschiedenen Ausführungsbeispielen wird ein in einer Nutzung einfaches Verfahren zum bereitgestellt, welches mit Hilfe von Fluoreszenz-Detektion und/oder durch Detektion von Absorption und/oder durch Detektion einer Oberflächenschädigung des Haars und Methoden aus der Prädiktiven Analytik eine präzise Ermittlung eines Grads einer Schädigung von Haar ermöglicht.

In verschiedenen Ausführungsbeispielen kann das Ermitteln des Haarzustands aufgrund seiner einfachen experimentellen Durchführbarkeit geeignet sein für eine Ausführung unter Verwendung einer tragbaren Datenverarbeitungsvorrichtung, auch als mobile Datenverarbeitungsvorrichtung bezeichnet. Als tragbare Datenverarbeitungsvorrichtung kann dabei beispielsweise ein Smartphone, ein iPad, ein Tablet oder Laptop genutzt werden.

In verschiedenen Ausführungsbeispielen kann ein Verfahren bereitgestellt werden, welches es ermöglicht, mittels einfacher bildanalytischer Verfahren, welche sich beispielsweise unter Verwendung einer mobilen Datenverarbeitungsvorrichtung (z.B. eines Smartphones), weniger weiterer einfacher Vorrichtungen (z.B. einer UV-LED, einer Weißlicht-, NIR- und/oder IR-Leuchtvorrichtung, eines Filters, eines tragbaren NIR-Sensors, eines tragbaren (NIR- und/oder VIS-) Spektrometers, eines Mikrofonkamms und/oder eines Mikroskops) und eines Prädiktive-Analytik-Verfahrens verwirklichen lassen, eine Empfehlung bezüglich der Haare des Nutzers, z.B. eines Haarbehandlungsmittels oder einer Haarbehandlung, bereitzustellen.

In verschiedenen Ausführungsbeispielen kann das empfohlene Haarbehandlungsmittel oder die empfohlene Haarbehandlung geeignet sein zum Erzielen einer gewünschten Wirkung (z.B. Haarfarbe, Haar-Pflegezustand, Haarstyling).

In verschiedenen Ausführungsbeispielen erlaubt die Verwendung der mathematischen Modelle aus dem Bereich Prädiktive Analytik (wie z.B. Tree Ensembles, neuronale Netze oder Support Vector Machines) eine wesentlich exaktere Berechnung der Schädigung (welche in den Modellen eine abhängige Variable bildet), als dies mit einfachen Modellen, z.B. einer einfachen linearen Regression, möglich wäre. Die Verfahren können dabei eine Vielzahl von Input-Variablen parallel nutzen und auch nicht-lineare Zusammenhänge abbilden. In verschiedenen Ausführungsbeispielen ermöglichen diese Modelle beispielsweise eine Einbeziehung kategorialer, nicht-metrischer Input-Variablen, wie z.B. einer Haarfarbe (z.B. blond, braun, schwarz, usw.) und/oder ethnischer Zugehörigkeit eines Haartyps (z.B. kaukasisch, asiatisch, afro-amerikanisch), welche einen Einfluss auf die gemessenen Werte haben können. Die Input-Variablen können in verschiedenen Ausführungsbeispielen mittels der Sensorvorrichtung und gegebenenfalls mittels der weiteren Sensorvorrichtung erfasst werden, z.B. die Haarfarbe unter Verwendung einer Kamera (z.B. der Smartphone-Kamera), eines Colorimeters oder einer Farbkarte, der Haartyp unter Verwendung der Kamera.

In verschiedenen Ausführungsbeispielen kann eine Kameraaufnahme des Haars genutzt werden, um mittels Bildanalyseverfahren nicht nur die Haarfarbe, sondern zusätzlich eine Haarstruktur zu ermitteln. Anhand einer Kombination von Haarfarbe und Haarstruktur kann gegebenenfalls die ethnische Zugehörigkeit, z.B. schwarz/kraus: afro-amerikanisch, schwarz-glatt: asiatisch, usw., ermittelt werden.

Gemäß verschiedenen Ausführungsformen kann die Mehrzahl von Messwert-beeinflussenden Parametern eine Haarfarbe und/oder eine ethnische Zugehörigkeit eines Typs des Haars aufweisen.

Gemäß verschiedenen Ausführungsformen kann die prädiktive Analytik mindestens ein Verfahren nutzen aus einer Gruppe von Verfahren, wobei die Gruppe von Verfahren aufweist:
lineare oder multi-lineare Regression, polynome Regression, neuronale-Netze-Verfahren, Support Vector Machine-Verfahren, Entscheidungsbäume-Verfahren ("Decision Trees", "Random Forest", "Tree Ensembles") und weitere Verfahren.

Mittels Verwendens verschiedener Sensoren wie z.B. Linsen, Gyroskope und Beschleunigungsmesser, kann es möglich sein, eine Position der Sensorvorrichtung oder der weiteren Sensorvorrichtung zu ermitteln, z.B. eine Position in einer Hand einer Person, um eine geeignete kosmetische Haarpflege (z.B. Produkte) zu ermitteln und einer unnötigen Haarschädigung, z.B. Haarverlust, vorzubeugen.

In verschiedenen Ausführungsbeispielen kann die Sensorvorrichtung oder die weitere Sensorvorrichtung einen Leitfähigkeitssensor zum Ermitteln einer Haarfeuchtigkeit aufweisen.

Die Datenübermittlung der Sensorvorrichtung zur Datenverarbeitungsvorrichtung und ggf. der weiteren Sensorvorrichtung zur Datenverarbeitungsvorrichtung kann in verschiedenen Ausführungsbeispielen mittels Kabel oder über bekannte Datenfunkverfahren und/oder -standards (z.B. Bluetooth, WLAN, NFC, ZigBee, Thread usw.) erfolgen.

Der Haarzustand weist mindestens einen Haarzustandsparameter auf (außer dem Cysteinsäuregehalt z.B. (oxidativer) Haarschädigungsgrad, Dehnungs-Haarschädigungsgrad, Haarfarbe, Haarfeuchtigkeit) und jeder von dem mindestens einen Sensorwert und dem mindestens einen weiteren Sensorwert ist geeignet zum Ermitteln mindestens eines der Haarzustandsparameter. Dabei kann jedem der Sensorwerte in verschiedenen Ausführungsbeispielen eine Zuverlässigkeitsmaßzahl zugeordnet sein.

In verschiedenen Ausführungsbeispielen kann in einem Fall, dass mindestens einer von dem mindestens einen Sensorwert und mindestens einer von dem mindestens einen weiteren Sensorwert geeignet sind zum Ermitteln desselben Haarzustandsparameters, z.B. des oxidativen Haarschädigungsgrads, das Ermitteln des Haarzustands ein Ermitteln des Haarzustandsparameters basierend auf einem anhand der Zuverlässigkeitsmaßzahlen gewichteten Mittelwert erfolgen. Damit kann sichergestellt sein, dass bei einem Ermitteln des Haarzustandsparameters basierend auf verschiedenen Sensoren oder Messverfahren dasjenige Messverfahren stärker gewichtet wird, das eine höhere Zuverlässigkeit aufweist.

In verschiedenen Ausführungsbeispielen kann anstelle eines gewichteten Mittelwerts nur das Messverfahren (der Sensor) mit der höheren Zuverlässigkeit ausgewertet werden.

In verschiedenen Ausführungsbeispielen kann die Sensorvorrichtung den NIR-Sensor aufweisen, und die weitere Sensorvorrichtung kann den beschriebenen Sensor zum Ermitteln des Haarschädigungsgrads mittels Fluoreszenzlicht aufweisen (d.h. die UV-Lichtquelle und die Kamera) und/oder den Kontaktmikrofon-Oberflächenrauhigkeitssensor und/oder die Kamera (z.B. zum Ermitteln der Haarfarbe) und/oder das Interferenzreflexionsmikroskop und/oder den Leitfähigkeitssensor, und/oder einen weiteren Sensor.

In verschiedenen Ausführungsbeispielen kann die Sensorvorrichtung den Sensor zum Ermitteln des Haarschädigungsgrads mittels Fluoreszenzlicht aufweisen, und die weitere Sensorvorrichtung kann den NIR-Sensor aufweisen und/oder das Kontaktmikrofon und/oder die Kamera (z.B. zum Ermitteln der Haarfarbe) und/oder das Interferenzreflexionsmikroskop und/oder den Leitfähigkeitssensor, und/oder einen weiteren Sensor.

In verschiedenen Ausführungsbeispielen kann die Sensorvorrichtung das Kontaktmikrofon aufweisen (z.B. als Sensor-Kamm gestaltet sein), und die weitere Sensorvorrichtung kann den NIR-Sensor aufweisen und/oder den Sensor zum Ermitteln des Haarschädigungsgrad mittels Fluoreszenzlicht und/oder die Kamera (z.B. zum Ermitteln der Haarfarbe) und/oder das Interferenzreflexionsmikroskop und/oder den Leitfähigkeitssensor, und/oder einen weiteren Sensor.

In verschiedenen Ausführungsbeispielen kann die Sensorvorrichtung das Interferenzreflexionsmikroskop aufweisen, und die weitere Sensorvorrichtung kann den (N)IR-Sensor aufweisen und/oder das Kontaktmikrofon und/oder die Kamera (z.B. zum Ermitteln der Haarfarbe) und/oder den Sensor zum Ermitteln des Haarschädigungsgrads mittels Fluoreszenzlicht und/oder den Leitfähigkeitssensor, und/oder einen weiteren Sensor.

In verschiedenen Ausführungsbeispielen kann die Sensorvorrichtung ferner mindestens einen weiteren Sensor aufweisen, beispielweise einen oder mehrere der beschriebenen Sensoren, die nicht Teil der zweiten Sensorvorrichtung sind, oder einen anderen Sensor.

Es werden ein System und ein Verfahren bereitgestellt, bei welchem eine Haarschädigung unter Einbeziehung verschiedener Analysemethoden gemessen wird. Ein Untersuchungsergebnis, z.B. ein ermittelter Haarschädigungsgrad, kann als eine Hilfestellung für eine passende Produktempfehlung und/oder Haarbehandlungsverfahrenempfehlung herangezogen werden.

In verschiedenen Ausführungsbeispielen kann das System einerseits die Haaroberfläche mittels eines (z.B. kleinen) Mikroskops analysieren, aber auch die Haarschädigung (in Form des Cysteinsäuregehalts) mittels der NIR-Messung ermitteln.

In verschiedenen Ausführungsbeispielen lassen sich verschiedene Analysemethoden vereinheitlichen und ein Haarzustand oder ein Haarzustandsparameter leichter und schneller ermitteln.

Erfindungsgemäß wird ein System zum Ermitteln eines Haarzustands bereitgestellt. Das System weist eine tragbare Sensorvorrichtung mit mindestens einem Sensor zum Erfassen mindestens eines Sensorwerts an Haaren eines Nutzers, eine weitere tragbare Sensorvorrichtung mit mindestens einem weiteren Sensor zum Erfassen des mindestens einen weiteren Sensorwerts an den Haaren des Nutzers und eine Datenverarbeitungsvorrichtung auf, wobei die Datenverarbeitungsvorrichtung eingerichtet ist ermitteln, ob der Datenverarbeitungsvorrichtung zusätzlich zum mindestens einen Sensorwert ferner des mindestens einen weiteren Sensorwerts von der weiteren Sensorvorrichtung bereitgestellt ist, und eingerichtet ist, den Haarzustand des Nutzers basierend auf dem erfassten mindestens einen Sensorwert zu ermitteln in einem Fall, dass ermittelt wurde, dass nur der mindestens eine Sensorwert bereitgestellt ist, oder basierend auf dem bereitgestellten mindestens einen Sensorwert und dem bereitgestellten mindestens einen weiteren Sensorwert in einem Fall, dass ermittelt wurde, dass ferner der mindestens eine weitere Sensorwert bereitgestellt ist, wobei der Haarzustand einen Haarzustandsparameter aufweist und jeder von dem mindestens einen Sensorwert und dem mindestens einen weiteren Sensorwert geeignet ist zum Ermitteln des Haarzustandsparameters, und wobei der Haarzustandsparameter einen Haarschädigungsgrad in Form eines Cysteinsäuregehalts aufweist.

In verschiedenen Ausführungsbeispielen kann mindestens einer von dem mindestens einen Sensor und dem mindestens einen weiteren Sensor einen optischen Sensor zum Ermitteln eines Cysteinsäuregehalts des Haars und/oder zum Ermitteln einer Haarfarbe des Nutzers aufweisen.

In verschiedenen Ausführungsbeispielen kann die Datenverarbeitungsvorrichtung Teil eines Smartphones, Tablets oder iPads sein.

In verschiedenen Ausführungsbeispielen können die Sensorvorrichtung und die Datenverarbeitungsvorrichtung eine integrierte tragbare Vorrichtung bilden.

Erfindungsgemäß wird ein Verfahren zum Ermitteln eines Haarzustands unter Verwendung des oben beschriebenen Systems bereitgestellt. Das Verfahren weist ein Erfassen mindestens eines Sensorwerts an Haaren eines Nutzers mittels der tragbaren Sensorvorrichtung auf, ein Bereitstellen des mindestens einen Sensorwerts an die Datenverarbeitungsvorrichtung, ein Ermitteln, mittels der Datenverarbeitungsvorrichtung, ob der Datenverarbeitungsvorrichtung ferner mindestens ein weiterer Sensorwert von der weiteren Sensorvorrichtung bereitgestellt ist, und ein Ermitteln eines Haarzustands des Nutzers mittels der Datenverarbeitungsvorrichtung basierend auf dem erfassten mindestens einen Sensorwert in einem Fall, dass ermittelt wurde, dass nur der mindestens eine Sensorwert bereitgestellt ist, oder basierend auf dem bereitgestellten mindestens einen Sensorwert und dem bereitgestellten mindestens einen weiteren Sensorwert in einem Fall, dass ermittelt wurde, dass ferner der mindestens eine weitere Sensorwert bereitgestellt ist, wobei der Haarzustand einen Haarzustandsparameter aufweist und jeder von dem mindestens einen Sensorwert und dem mindestens einen weiteren Sensorwert geeignet ist zum Ermitteln des Haarzustandsparameters, und wobei der Haarzustandsparameter einen Haarschädigungsgrad in Form eines Cysteinsäuregehalts aufweist.

In verschiedenen Ausführungsbeispielen kann das Ermitteln des Schädigungsgrads des Haars ein Ermitteln einer Oberflächenrauhigkeit des Haars aufweisen.

In verschiedenen Ausführungsbeispielen kann das Ermitteln eines Haarzustands ein Ermitteln einer Haarfeuchtigkeit des Nutzers aufweisen.

In verschiedenen Ausführungsbeispielen kann das Ermitteln eines Haarzustands ein Ermitteln einer Haarfarbe des Nutzers aufweisen.

In verschiedenen Ausführungsbeispielen kann der Haarzustand mindestens einen Haarzustandsparameter aufweisen und jeder von dem mindestens einen Sensorwert und dem mindestens einen weiteren Sensorwert kann geeignet sein zum Ermitteln mindestens eines der Haarzustandsparameter, und jedem der Sensorwerte kann eine Zuverlässigkeitsmaßzahl zugeordnet sein.

In verschiedenen Ausführungsbeispielen kann in einem Fall, dass mindestens einer von dem mindestens einen Sensorwert und mindestens einer von dem mindestens einen weiteren Sensorwert geeignet sind zum Ermitteln desselben Haarzustandsparameters, das Ermitteln des Haarzustands ein Ermitteln des Haarzustandsparameters basierend auf einem anhand der Zuverlässigkeitsmaßzahlen gewichteten Mittelwert erfolgen.

In verschiedenen Ausführungsbeispielen wird ein Verfahren zum Ermitteln einer Empfehlung bezüglich Haaren eines Nutzers bereitgestellt. Das Verfahren kann ein Ermitteln eines Haarzustands gemäß verschiedenen Ausführungsbeispielen und ein Ermitteln einer Empfehlung bezüglich Haaren des Nutzers basierend auf dem ermittelten Haarzustand aufweisen.

In verschiedenen Ausführungsbeispielen kann die Empfehlung bezüglich Haaren eines Nutzers mindestens eine aufweisen von einer Haarbehandlungsproduktempfehlung und einer Haarbehandlungsverfahrenempfehlung.

Ausführungsbeispiele der Erfindung sind in den Figuren dargestellt und werden im Folgenden näher erläutert.

Es zeigen
Figur 1A, 1B und 1C zeigen jeweils eine schematische Darstellung eines Systems zum Ermitteln eines Haarzustands gemäß verschiedenen Ausführungsbeispielen;
Figur 2 ein Ablaufdiagramm, welches ein Verfahren zum Ermitteln eines Haarzustands gemäß verschiedenen Ausführungsbeispielen darstellt; und
Figur 3 ein Ablaufdiagramm, welches ein Verfahren zum Ermitteln einer Empfehlung bezüglich Haaren eines Nutzers gemäß verschiedenen Ausführungsbeispielen darstellt.

In der folgenden ausführlichen Beschreibung wird auf die beigefügten Zeichnungen Bezug genommen, die Teil der vorliegenden Anmeldung bilden und in denen zur Veranschaulichung spezifische Ausführungsformen gezeigt sind, in denen die Erfindung ausgeübt werden kann. Es versteht sich, dass andere Ausführungsformen benutzt und strukturelle oder logische Änderungen vorgenommen werden können, ohne von dem Schutzumfang der vorliegenden Erfindung abzuweichen. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche definiert.

In der vorliegenden Beschreibung werden die Begriffe Prädiktive Analytik, Predictive Analytics, Big Data und Data Mining synonym verwendet.

Bei einem hierin genannten Smartphone ist dies, sofern aus dem Kontext nicht etwas Anderes hervorgeht, als stellvertretend zu verstehen für alle ähnlichen von tragbarer Datenverarbeitungsvorrichtung, d.h. Smartphones, Tablets, iPads, Laptops, usw. Sinngemäßes gilt für Smartphonekameras und Ähnliches.

Unter einem Haarfärbemittel ist hierin ein **Mittel** zum Ändern einer Haarfarbe zu verstehen. Das Haarfärbemittel kann also entweder eine Coloration zum Erzeugen einer Haarfarbe (z.B. schwarz, braun rot) sein, oder ein Blondierungsmittel zum Aufhellen einer Haarfarbe.

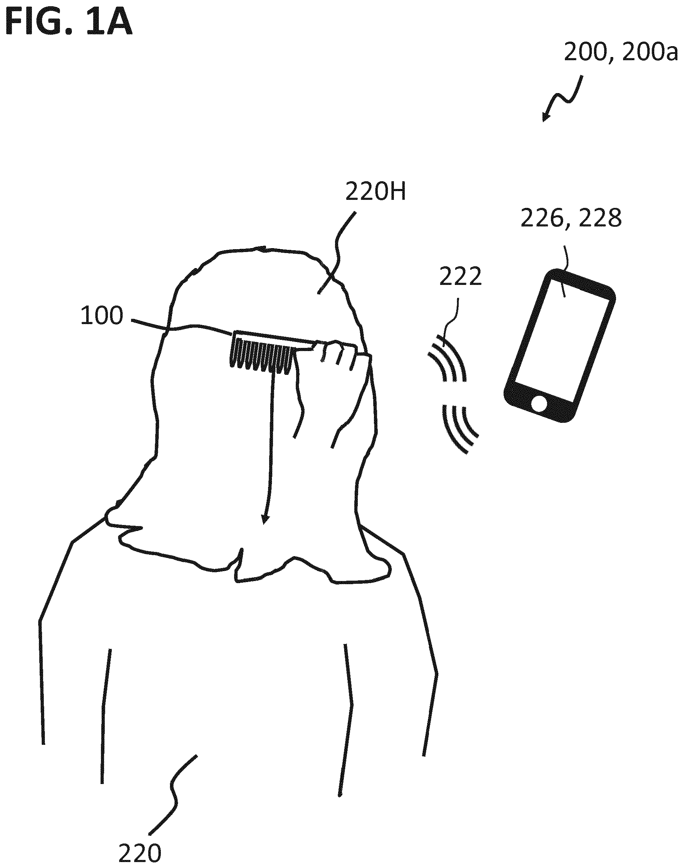

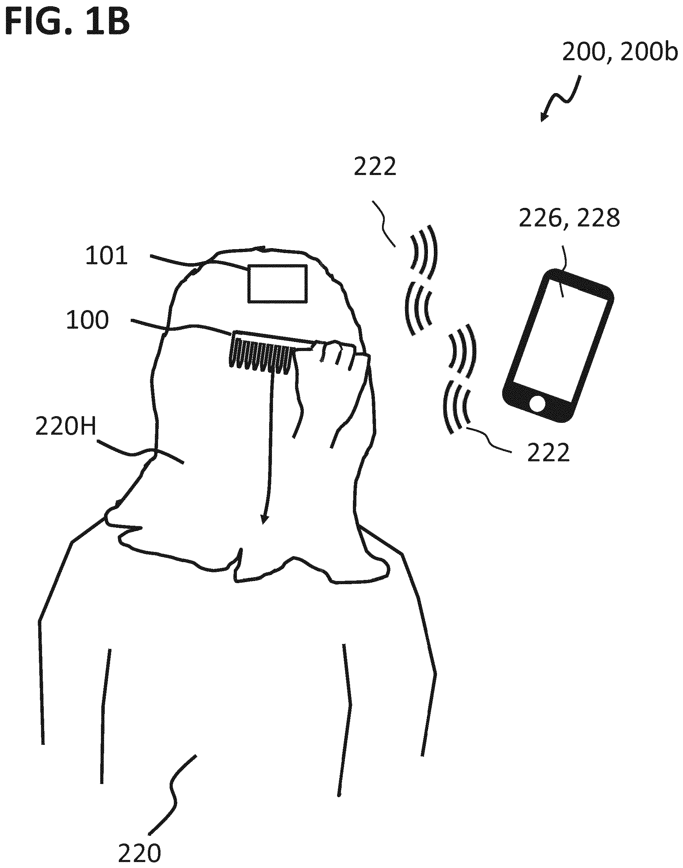

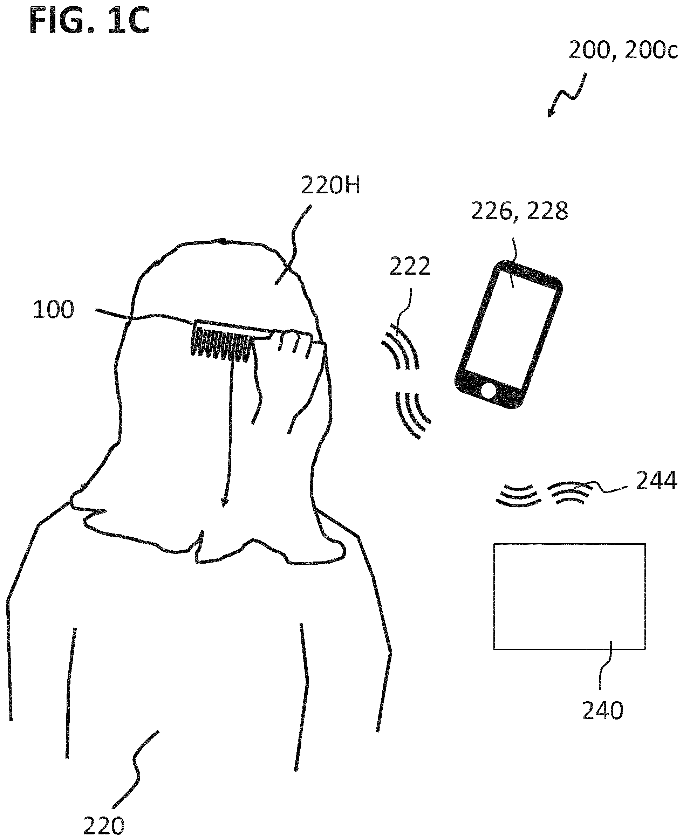

FIG. 1A, 1 B und 1C zeigen jeweils eine schematische Darstellung eines Systems zum Ermitteln eines Haarzustands gemäß verschiedenen Ausführungsbeispielen.

Unterschiedliche Ausführungsbeispiele des Systems 200 zum Ermitteln eines Haarzustands sind mit nachgestellten Buchstaben gekennzeichnet.

Das System 200 weist eine tragbare Sensorvorrichtung 100 mit mindestens einem Sensor zum Erfassen mindestens eines Sensorwerts an Haaren 220H eines Nutzers 220 auf.

Das System 200 weist ferner eine Datenverarbeitungsvorrichtung 226 auf.

Die Datenverarbeitungsvorrichtung 226 ist eingerichtet zu ermitteln, ob der Datenverarbeitungsvorrichtung 226 zusätzlich zum mindestens einen Sensorwert (von der Sensorvorrichtung 100) ferner mindestens ein weiterer Sensorwert von einer weiteren Sensorvorrichtung 101 (siehe FIG. 1B) bereitgestellt ist. Die Datenverarbeitungsvorrichtung 226 ist eingerichtet, den Haarzustand des Nutzers 220 basierend auf dem erfassten mindestens einen Sensorwert zu ermitteln in einem Fall, dass ermittelt wurde, dass nur der mindestens eine Sensorwert bereitgestellt ist, oder basierend auf dem bereitgestellten mindestens einen Sensorwert und dem bereitgestellten mindestens einen weiteren Sensorwert zu ermitteln in einem Fall, dass ermittelt wurde, dass ferner der mindestens eine weitere Sensorwert bereitgestellt ist.

Anders ausgedrückt kann das System 200 in verschiedenen Ausführungsbeispielen als ein modulares System bereitgestellt sein, bei welchem die Sensorvorrichtung 100 und eine weitere Sensorvorrichtung 101 ausreichend sein können, um den Haarzustand des Nutzers 220 zu ermitteln, aber außerdem eine oder mehrere weitere Sensorvorrichtung(en) 101, die jeweils einen oder mehrere weitere Sensoren aufweisen können, in das Ermitteln des Haarzustands einbeziehbar sind, beispielsweise um zusätzlich zu einem oder mehreren Haarzustandsparameter, mittels welcher der Haarzustand beschrieben werden kann und welche(r) mittels der Sensorvorrichtung 100 ermittelbar sind, weitere Haarzustandsparameter zu ermitteln, und/oder um eine Genauigkeit des mittels der Sensorvorrichtung 100 ermittelten Haarzustandsparameters zu verbessern.

Außerdem ermöglicht der modulare Aufbau des Systems 200 dem Nutzer 220, zunächst ein relativ kostengünstiges Basissystem zu erwerben (wie z.B. in FIG. 1A dargestellt), und dieses später mittels einer oder mehrerer weiterer tragbarer Sensorvorrichtung(en) 101 zu erweitern (wie in FIG. 1B dargestellt).

In verschiedenen Ausführungsbeispielen kann das System zum Ermitteln des Haarzustands eine Ausgabevorrichtung 228, z.B. eine Anzeigevorrichtung (z.B. ein Bildschirm und/oder ein Mikrofon eines Smartphones) aufweisen.

In verschiedenen Ausführungsbeispielen kann das System zum Ermitteln des Haarzustands eine Eingabevorrichtung, z.B. einen berührungsempfindlichen Bildschirm 228 eines Smartphones, aufweisen.

In verschiedenen Ausführungsbeispielen kann das Ermitteln des Haarzustands, wie oben beschrieben ein Ermitteln eines Schädigungsgrads des Haars 220H (beispielsweise als oxidativen Haarschädigungsgrad und/oder als mechanischen (z.B. Dehnungs-)Haarschädigungsgrad), einer Haarfarbe, einer Haarfeuchtigkeit, oder anderer oder weiterer Haarzustandsparameter aufweisen.

In verschiedenen Ausführungsbeispielen kann der mindestens eine Sensor (der Sensorvorrichtung 100) einen oder mehrere der oben beschriebenen Sensoren aufweisen, z.B. einen (N)IR-Sensor, einen oben beschriebenen Sensor zum Ermitteln des Haarschädigungsgrads mittels Fluoreszenzlicht (d.h. mit UV-Lichtquelle Kamera), ein Kontaktmikrofon(zum Beipsiel zum Ermitteln der Oberflächenrauhigkeit), eine Kamera (z.B. zum Ermitteln der Haarfarbe), ein Interferenzreflexionsmikroskop, einen Leitfähigkeitssensor und/oder einen weiteren Sensor.

In verschiedenen Ausführungsbeispielen kann der mindestens eine weitere Sensor (der weiteren Sensorvorrichtung 101) einen oder mehrere der oben beschriebenen Sensoren aufweisen, z.B. einen NIR-Sensor, einen oben beschriebenen Sensor zum Ermitteln des Haarschädigungsgrads mittels Fluoreszenzlicht (d.h. mit UV-Lichtquelle Kamera), einen Kontaktmikrofon-Oberflächenrauhigkeitssensor, eine Kamera (z.B. zum Ermitteln der Haarfarbe), ein Interferenzreflexionsmikroskop, einen Leitfähigkeitssensor und/oder einen weiteren Sensor.

Der Sensor oder der weitere Sensor zum Ermitteln eines Cysteinsäuregehalts des Haars kann, wie oben beschrieben, in verschiedenen Ausführungsbeispielen eine Kamera und/oder ein Spektrometer zum Erfassen von Licht in einem Wellenlängenbereich sichtbaren Lichts aufweisen, wobei das Licht von dem Haar bei einem Bestrahlen mit UV-Licht als Fluoreszenzlicht abgestrahlt wird.

Bei dem Sensor oder dem weiteren Sensor kann in verschiedenen Ausführungsbeispielen ein einzelner Sensor derart eingerichtet sein, dass nur das Fluoreszenzlicht auswertbar erfasst wird, beispielsweise in einem Fall, dass zwischen dem Haar und dem optischen Sensor mindestens ein Filter angeordnet ist, welcher oder von welchen mindestens einer nur oder hauptsächlich einen Wellenlängenbereich des Fluoreszenzlichts passieren lässt.

Sofern vorgesehen ist, in einem solchen Fall zusätzlich die Haarfarbe mittels der Sensorvorrichtung 100 oder 101 zu erfassen, kann der Filter entfernbar eingerichtet sein, beispielsweise als ein Filteraufsatz für eine Smartphonekamera, so dass das Erfassen des Fluoreszenzlichts und ein Erfassen sichtbaren Lichts in mehreren Wellenlängenbereichen (z.B. R, G, B) zum Ermitteln der Haarfarbe sequenziell erfolgen kann. Alternativ kann der Filter mehrere Bereiche aufweisen, beispielsweise einen Bandpassbereich für das Fluoreszenzlicht, ggf. einen Bandpassbereich außerhalb des Fluoreszenzlicht-Wellenlängenbereichs und/oder einen im Wesentlichen ungefilterten Bereich. Alternativ kann der mindestens eine Sensor oder weitere Sensor eine Mehrzahl von Sensoren aufweisen, wobei mindestens einer der Sensoren zum Erfassen des Fluoreszenzlichts genutzt wird, und ein weiterer der Sensoren zum Ermitteln der Haarfarbe eingerichtet ist, beispielsweise als Kamera, z.B. eine Kamera eines Smartphones, Tablets, usw., oder z.B. als Spektrometer.

Bei dem Sensor oder dem weiteren Sensor kann in verschiedenen Ausführungsbeispielen ein einzelner Sensor derart eingerichtet sein, dass sowohl das Fluoreszenzlicht auswertbar erfasst wird, als auch ein Ermitteln der Haarfarbe des Nutzers ermöglicht ist, beispielsweise bei einer Gestaltung des Sensors Sensor oder des weiteren Sensor als Spektrometer.

Der Sensor oder der weitere Sensor zum Ermitteln eines Cysteinsäuregehalts des Haars kann, wie oben beschrieben, in verschiedenen Ausführungsbeispielen eine (N)IR-Kamera und/oder ein (N)IR-Spektrometer zum Erfassen von Licht in einem Wellenlängenbereich aufweisen, in welchem die Cysteinsäure Licht absorbiert.

Sofern vorgesehen ist, in einem solchen Fall zusätzlich die Haarfarbe mittels der Sensorvorrichtung 100 oder 101 zu erfassen, kann die Sensorvorrichtung 100 oder 101 einen weiteren der Sensor zum Erfassen des sichtbaren Lichts zum Ermitteln der Haarfarbe aufweisen.

In verschiedenen Ausführungsbeispielen können der mindestens eine Sensor der Sensorvorrichtung 100 und der mindestens eine weitere Sensor der weiteren Sensorvorrichtung 101 unterschiedliche Sensoren sein.

In verschiedenen Ausführungsbeispielen kann die Datenverarbeitungsvorrichtung 226 Teil eines Smartphones, Tablets oder Laptops sein, wie beispielhaft in FIG. 1A, 1B und 1C dargestellt ist.

In verschiedenen Ausführungsbeispielen können die Sensorvorrichtung 100 und die Datenverarbeitungsvorrichtung 226 eine integrierte tragbare Vorrichtung bilden, beispielsweise in einem Fall, dass der Sensor Teil der Datenverarbeitungsvorrichtung 226 ist, z.B. bei Nutzung einer Kamera und/oder eines Mikrofon(aufsatze)s eines Smartphones als den Sensor.

In verschiedenen Ausführungsbeispielen kann oder können das registrierte Licht und/oder das registrierte Geräusch oder ggf. ein anderer oder weiterer erfasster Sensorwert als Signal 222, z.B. als Rohdaten und/oder in verarbeiteter Form, beispielsweise als Digitalfoto oder eine andere Quantifizierung des registrierten Lichts, als Audiodatei, Fouriertransformation o.ä. an die Datenverarbeitungsvorrichtung 226 übertragen werden.

Die Übertragung kann in verschiedenen Ausführungsbeispielen auf bekanntem Weg erfolgen, beispielsweise mittels eines Datenkabels, kabelloser Datenübertragung (z.B. Bluetooth oder Near Field Communication (NFC)), oder eine Übertragung kann innerhalb einer Vorrichtung erfolgen, wenn die Sensorvorrichtung 100, wie oben beschrieben, mit der Datenverarbeitungsvorrichtung 226 die integrierte Vorrichtung bildet (z.B. bei Verwendung der Kamera und/oder des Mikrofons eines Smartphones, Tablets, Laptops o.ä. als Kamera).

In verschiedenen Ausführungsbeispielen kann die Sensorvorrichtung 100, wie in FIG. 1A, 1B und 1C beispielshaft dargestellt, als Kamm oder Bürste gestaltet sein. Anders ausgedrückt kann der mindestens eine Sensor in einen kamm- oder bürstenförmigen Körper integriert sein.

In verschiedenen Ausführungsbeispielen kann insbesondere das Mikrofon in den kamm- oder bürstenförmigen Körper integriert sein, beispielsweise wie oben beschrieben. In verschiedenen Ausführungsbeispielen kann sich das Mikrofon an/in Zähnen oder an/in den Borsten des kamm-oder bürstenförmigen Körpers befinden.

In verschiedenen Ausführungsbeispielen können ein oder mehrere Sensoren alternativ oder zusätzlich in den kamm- oder bürstenförmigen Körper integriert sein.

In verschiedenen Ausführungsbeispielen kann die Sensorvorrichtung 100 oder die weitere Sensorvorrichtung 101 einen, sofern zweckmäßig, im Wesentlichen beliebig geformten Körper aufweisen.

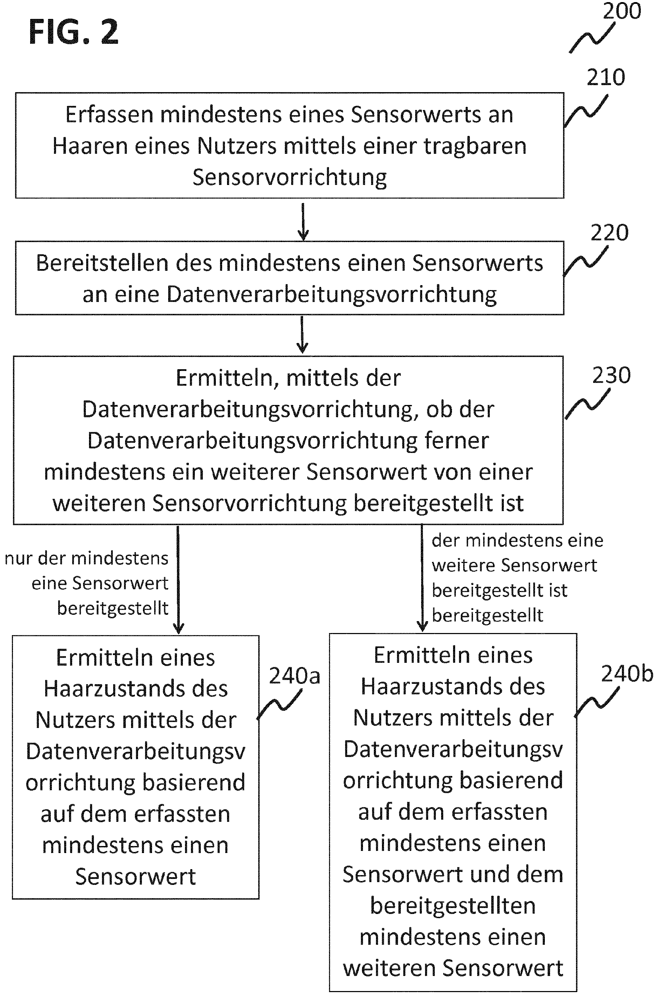

In verschiedenen Ausführungsbeispielen, wie z.B. in FIG. 2A bis FIG. 2K dargestellt, können die Sensorvorrichtung 108 und die Datenverarbeitungsvorrichtung 116 eine integrierte Vorrichtung bilden.

In verschiedenen Ausführungsbeispielen können die Sensorvorrichtung 100 oder die weitere Sensorvorrichtung 101 und/oder die Datenverarbeitungsvorrichtung 226 eine Vorrichtung zur kabellosen Datenübertragung aufweisen, beispielsweise für eine Datenübertragung mittels WLAN, Bluetooth, Thread, ZigBee, NFC oder ähnliches, z.B. wie oben beschrieben.

Zum Empfangen und Weiterverarbeiten der Signale/Daten kann die Datenverarbeitungsvorrichtung 226 in verschiedenen Ausführungsbeispielen mit einer entsprechenden Software ausgestattet sein, beispielsweise einer App, beispielsweise wie oben beschrieben. Dabei kann eine einzelne Software/App bereitgestellt sein, um den Haarzustand auf Grundlage der Sensorwerte von der Sensorvorrichtung 100 zu ermitteln und um den Haarzustand auf Grundlage der Sensorwerte von der Sensorvorrichtung und der weiteren Sensorvorrichtung 101 zu ermitteln.

In verschiedenen Ausführungsbeispielen kann mittels der Datenverarbeitungsvorrichtung 226 anhand der ermittelten Lichtintensität (beispielsweise Fluoreszenzlicht oder (N)IR-Licht) ein Cysteinsäuregehalt ermittelt werden. Hierfür können, z.B. wie oben beschrieben, mathematische Modelle aus dem Bereich Prädiktive Analytik genutzt werden, um eine Beziehung zwischen der (standardisierten) Lichtintensität und einem zugehörigen Cysteinsäuregehalt (und damit einem Schädigungsgrad des Haars) zu ermitteln.

Als unabhängige Parameter können in das Modell dabei in verschiedenen Ausführungsbeispielen beispielsweise eine Beziehung zwischen Cysteinsäuregehalt und Lichtintensität oder entsprechende Datenwerte, z.B. in Form zugeordneter Datenpaare, einbezogen werden, welche mittels Vermessung von Standard-Haarproben, welche einen anhand bekannter aufwändiger Verfahren ermittelten Cysteinsäuregehalt aufweisen, ermittelt oder mathematisch modelliert wurden.

Entsprechend kann in verschiedenen Ausführungsbeispielen mit anderen Sensorwerten verfahren werden, um den Schädigungsgrad des Haars und/oder die Haarfarbe zu ermitteln.

In verschiedenen Ausführungsbeispielen kann der Schädigungsgrad des Haars in einer kategorialen Skala (z.B. leicht, mittel, schwer) ermittelt werden.

In verschiedenen Ausführungsbeispielen kann der Schädigungsgrad in einer metrischen Skala (z.B. Prozentanteil des Gehalts an Cysteinsäure, Prozentanteil an Flächen mit höherer Interferenz o.ä.) ermittelt werden.

Die Datenverarbeitungsvorrichtung 226 kann, wie oben beschrieben, eine mobile Datenverarbeitungsvorrichtung, beispielsweise ein Smartphone, ein Tablet oder einen Laptop, aufweisen, insbesondere in einem Fall, dass die tragbare Sensorvorrichtung 100 mit der Datenverarbeitungsvorrichtung 226 die integrierte Vorrichtung bildet.

In verschiedenen Ausführungsbeispielen kann die Datenverarbeitungsvorrichtung 226 von anderer Art sein, z.B. ein Desktop-Computer, integriert in einen Smart Mirror, oder jede andere Datenverarbeitungsvorrichtung 226, die geeignet ist, die Daten zu speichern und bereitzustellen und das Prädiktive Analytik Verfahren auszuführen, also beispielsweise jede Datenverarbeitungsvorrichtung 226 mit hinreichend großem Datenspeicher und hinreichend leistungsfähigem Prozessor.

In verschiedenen Ausführungsbeispielen können anstelle des Prädiktive-Analytik-Verfahrens andere, z.B. einfachere, Verfahren zum Ermitteln des Haarzustands, z.B. des Schädigungsgrads, der Haarfarbe und/oder der Haarfeuchtigkeit, genutzt werden. Beispielhaft seien Zuordnungsvorschriften erwähnt.

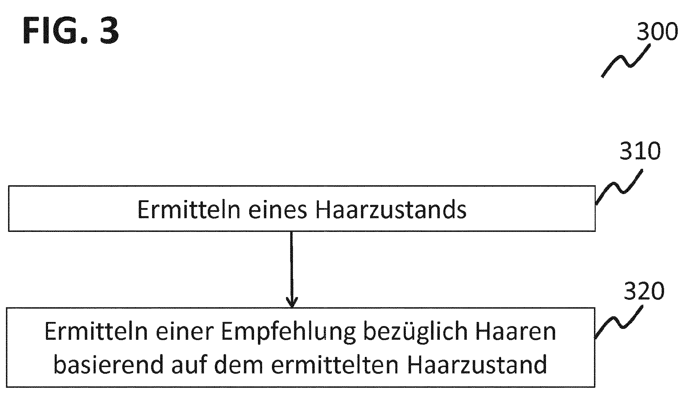

In verschiedenen Ausführungsbeispielen, wie in FIG. 3C dargestellt, kann die Datenverarbeitungsvorrichtung 226 eingerichtet sein, den Haarzustand, z.B. den Haarschädigungsgrad und/oder die Haarfarbe, und/oder einer Empfehlung basierend auf dem Haarzustand indirekt zu ermitteln, beispielsweise mittels eines Übermittelns eines Signals 244 (z.B. der Sensor-Rohdaten und/oder von teilweise ausgewerteten Sensordaten und/oder des ermittelten Haarzustands) an eine externe Datenverarbeitungsvorrichtung 240, beispielsweise an eine Cloud-Prozessorarchitektur (kurz: Cloud), und mittels Empfangens eines Ergebnisses von der externen Datenverarbeitungsvorrichtung 240 (z.B. der Cloud).

In verschiedenen Ausführungsbeispielen kann die Vorrichtung zum Bereitstellen des Haarbehandlungsmittels als ein lernendes System gestaltet sein, beispielsweise indem der Nutzer und/oder weitere Nutzer den Haarzustand vor einer Anwendung des Haarbehandlungsmittels und den Haarzustand nach einem Behandeln des Haars mit dem Haarbehandlungsmittel der Datenverarbeitungsvorrichtung 226 (beispielsweise mittels der Cloud) bereitstellt/bereitstellen.

Zum Bereitstellen des Haarzustands nach der Behandlung kann in verschiedenen Ausführungsbeispielen ein Ermitteln des Haarzustands nach dem Behandeln mit dem Haarbehandlungsmittel mittels des Systems 200 zum Ermitteln des Haarzustands genutzt werden.

FIG. 2 zeigt ein Ablaufdiagramm 200, welches ein Verfahren zum Ermitteln eines Haarzustands gemäß verschiedenen Ausführungsbeispielen darstellt. Zum Ausführen des Verfahrens kann eine Vorrichtung gemäß verschiedenen Ausführungsbeispielen wie oben beschrieben verwendet werden.

Das Verfahren weist auf ein Erfassen mindestens eines Sensorwerts an Haaren eines Nutzers mittels einer tragbaren Sensorvorrichtung (bei 210), ein Bereitstellen des mindestens einen Sensorwerts an eine Datenverarbeitungsvorrichtung (bei 220), ein Ermitteln, mittels der Datenverarbeitungsvorrichtung, ob der Datenverarbeitungsvorrichtung ferner mindestens ein weiterer Sensorwert von einer weiteren Sensorvorrichtung bereitgestellt ist (bei 230) und entweder ein Ermitteln eines Haarzustands des Nutzers mittels der Datenverarbeitungsvorrichtung basierend auf dem erfassten mindestens einen Sensorwert (bei 240a, in einem Fall, dass nur der mindestens eine Sensorwert bereitgestellt ist) oder ein Ermitteln eines Haarzustands des Nutzers mittels der Datenverarbeitungsvorrichtung basierend auf dem erfassten mindestens einen Sensorwert und dem bereitgestellten mindestens einen weiteren Sensorwert (bei 240b, in einem Fall, dass der mindestens eine weitere Sensorwert bereitgestellt bereitgestellt ist).

FIG. 3 zeigt ein Ablaufdiagramm 300, welches ein Verfahren zum Ermitteln einer Empfehlung bezüglich Haaren eines Nutzers gemäß verschiedenen Ausführungsbeispielen darstellt.

Das Verfahren kann ein Ermitteln eines Haarzustands gemäß verschiedenen Ausführungsbeispielen aufweisen (bei 310), und ein Ermitteln einer Empfehlung bezüglich Haaren basierend auf dem ermittelten Haarzustand (bei 320).

Dabei kann die Empfehlung in verschiedenen Ausführungsbeispielen eine Produktempfehlung und/oder eine Haarbehandlungsempfehlung sein.

Das Ermitteln des Haarzustands weist zumindest ein

Ermitteln eines Schädigungsgrads des Haars auf.

Weitere vorteilhafte Ausgestaltungen des Verfahrens ergeben sich aus der Beschreibung der Vorrichtung und umgekehrt.

## Patentansprüche

1. System (200) zum Ermitteln eines Haarzustands, aufweisend:
eine tragbare Sensorvorrichtung (100) mit mindestens einem Sensor zum Erfassen mindestens
eines Sensorwerts an Haaren eines Nutzers;
eine weitere tragbare Sensorvorrichtung (101) mit mindestens einem weiteren Sensor zum Erfassen mindestens eines weiteren Sensorwerts an den Haaren des Nutzers; und
eine Datenverarbeitungsvorrichtung (226), wobei die Datenverarbeitungsvorrichtung (226) eingerichtet ist zu ermitteln, ob der Datenverarbeitungsvorrichtung (226) zusätzlich zum mindestens einen Sensorwert ferner der mindestens eine weitere Sensorwert von der weiteren Sensorvorrichtung (101) bereitgestellt ist, und eingerichtet ist, den Haarzustand des Nutzers basierend auf dem erfassten mindestens einen Sensorwert zu ermitteln in einem Fall, dass ermittelt wurde, dass nur der mindestens eine Sensorwert bereitgestellt ist, oder basierend auf dem bereitgestellten mindestens einen Sensorwert und dem bereitgestellten mindestens einen weiteren Sensorwert in einem Fall, dass ermittelt wurde, dass ferner der mindestens eine weitere Sensorwert bereitgestellt ist,
wobei der Haarzustand einen Haarzustandsparameter aufweist und jeder von dem mindestens einen Sensorwert und dem mindestens einen weiteren Sensorwert geeignet ist zum Ermitteln des Haarzustandsparameters, und
wobei der Haarzustandsparameter einen Haarschädigungsgrad in Form eines Cysteinsäuregehalts aufweist.

2. System gemäß Anspruch 1,
wobei mindestens einer von dem mindestens einen Sensor und dem mindestens einen weiteren Sensor einen optischen Sensor zum Ermitteln eines Cysteinsäuregehalts des Haars und/oder zum Ermitteln einer Haarfarbe des Nutzers aufweist.

3. System gemäß einem der vorhergehenden Ansprüche,
wobei einer von dem mindestens einen Sensor und dem mindestens einen weiteren Sensor ein Mikrofon zum Ermitteln einer Oberflächenrauhigkeit des Haars aufweist.

4. System gemäß einem der vorhergehenden Ansprüche,
wobei die Datenverarbeitungsvorrichtung Teil eines Smartphones, Tablets oder iPads ist.

5. System gemäß einem der vorhergehenden Ansprüche,
wobei die erste Sensorvorrichtung und die Datenverarbeitungsvorrichtung eine integrierte tragbare Vorrichtung bilden.

6. Verfahren zum Ermitteln eines Haarzustands unter Verwendung eines Systems gemäß
einem der vorhergehenden Ansprüche, aufweisend:
Erfassen (210) mindestens eines Sensorwerts an Haaren eines Nutzers mittels der tragbaren Sensorvorrichtung (100);
Bereitstellen (220) des mindestens einen Sensorwerts an die Datenverarbeitungsvorrichtung (226);
Ermitteln (230), mittels der Datenverarbeitungsvorrichtung (226), ob der Datenverarbeitungsvorrichtung ferner mindestens ein weiterer Sensorwert von der weiteren Sensorvorrichtung (101) bereitgestellt ist;
Ermitteln (240a,b) eines Haarzustands des Nutzers mittels der Datenverarbeitungsvorrichtung basierend auf dem erfassten mindestens einen Sensorwert in einem Fall, dass ermittelt wurde, dass nur der mindestens eine Sensorwert bereitgestellt ist, oder basierend auf dem bereitgestellten mindestens einen Sensorwert und dem bereitgestellten mindestens einen weiteren Sensorwert in einem Fall, dass ermittelt wurde, dass ferner der mindestens eine weitere Sensorwert bereitgestellt ist,
wobei der Haarzustand einen Haarzustandsparameter aufweist und jeder von dem mindestens einen Sensorwert und dem mindestens einen weiteren Sensorwert geeignet ist zum Ermitteln des Haarzustandsparameters, und
wobei der Haarzustandsparameter einen Haarschädigungsgrad in Form eines Cysteinsäuregehalts aufweist.

7. Verfahren gemäß Anspruch 6,
wobei das Ermitteln eines Haarzustands ein Ermitteln einer Haarfeuchtigkeit des Nutzers aufweist.

8. Verfahren zum Ermitteln einer Empfehlung bezüglich Haaren eines Nutzers,
aufweisend:
Ermitteln eines Haarzustands gemäß einem der Ansprüche 6 oder 7; und
Ermitteln einer Empfehlung bezüglich Haaren des Nutzers basierend auf dem ermittelten Haarzustand.

9. Verfahren gemäß Anspruch 8,
wobei die Empfehlung bezüglich Haaren eines Nutzers mindestens eine aufweist von einer Haarbehandlungsproduktempfehlung und einer Haarbehandlungsverfahrenempfehlung.

10. Verfahren gemäß Anspruch 8 oder 9,
wobei dem Nutzer ermöglicht wird, das empfohlene Haarbehandlungsprodukt online zu bestellen und/oder dem Nutzer angegeben wird, wo das empfohlene Haarbehandlungsprodukt erhältlich ist.

## Claims

1. A system (200) for determining a hair condition, comprising:
a portable sensor device (100) with at least one sensor for detecting at least one sensor value on a user's hair;
a further portable sensor device (101) with at least one further sensor for detecting the at least one further sensor value on the user's hair; and a data processing device (226), wherein the data processing device (226) is configured to determine whether, in addition to the at least one sensor value, the at least one further sensor value is also provided by the further sensor device (101), and configured to determine the hair condition of the user based on the detected at least one sensor value in a case where it has been determined that only the at least one sensor value is provided, or based on the provided at least one sensor value and the provided at least one further sensor value in a case where it has been determined that the at least one further sensor value is also provided,
wherein the hair condition has a hair condition parameter and each of the at least one sensor value and the at least one further sensor value is suitable for determining the hair condition parameter, and
wherein the hair condition parameter comprises a degree of hair damage in the form of a cystine acid content.

2. System according to claim 1,
wherein at least one of the at least one sensor and the at least one further sensor comprises an optical sensor for determining a cysteine acid content of the hair and/or for determining a hair color of the user.

3. System according to one of the preceding claims,
wherein one of the at least one sensor and the at least one further sensor comprises a microphone for determining a surface roughness of the hair.

4. System according to one of the preceding claims,
wherein the data processing device is part of a smartphone, tablet, or iPad.

5. System according to one of the preceding claims,
wherein the first sensor device and the data processing device form an integrated portable device.

6. Method for determining a hair condition using a system according to one of the
preceding claims, comprising:
Sensing (210) at least one sensor value on a user's hair using the portable sensor device;
Providing (220) the at least one sensor value to the data processing device (226);
determining (230), by means of the data processing device (226), whether the data processing device is also provided with at least one further sensor value from the further sensor device (101);
determining (240a,b) a hair condition of the user using the data processing device based on the acquired at least one sensor value in a case where it has been determined that only the at least one sensor value is provided, or based on the provided at least one sensor value and the provided at least one further sensor value in a case where it has been determined that the at least one further sensor value is also provided,
wherein the hair condition has a hair condition parameter and each of the at least one sensor value and the at least one further sensor value is suitable for determining the hair condition parameter, and
wherein the hair condition parameter comprises a degree of hair damage in the form of a cystine acid content.

7. Method according to claim 6,
wherein determining a hair condition comprises determining a hair moisture level of the user.

8. A method for determining a recommendation regarding a user's hair,
comprising:
determining a hair condition according to one of claims 6 or 7; and
determining a recommendation regarding the user's hair based on the determined hair condition.

9. Method according to claim 8,
wherein the recommendation regarding a user's hair comprises at least one of a hair treatment product recommendation and a hair treatment method recommendation.

10. Method according to claim 8 or 9,
whereby the user is enabled to order the recommended hair treatment product online and/or the user is informed where the recommended hair treatment product is available.

## Revendications

1. Système (200) permettant de déterminer l'état des cheveux, comprenant :
un dispositif capteur portable (100) comprenant au moins un capteur pour détecter au moins une valeur de capteur sur les cheveux d'un utilisateur ;
un autre dispositif de détection portable (101) comprenant au moins un autre capteur pour détecter au moins une autre valeur de capteur sur les cheveux de l'utilisateur ; et un dispositif de traitement de données (226), le dispositif de traitement de données (226) étant configuré pour déterminer si, en plus de la au moins une valeur de capteur, le dispositif de détection supplémentaire fournit également la au moins une autre valeur de capteur provenant du dispositif de détection supplémentaire (101), et est conçu pour déterminer l'état des cheveux de l'utilisateur sur la base de la au moins une valeur de capteur détectée dans le cas où il a été déterminé que seule la au moins une valeur de capteur est fournie, ou sur la base de la au moins une valeur de capteur fournie et de la au moins une autre valeur de capteur fournie dans le cas où il a été déterminé que la au moins une autre valeur de capteur est également fournie, l'état des cheveux présentant un paramètre d'état des cheveux et chacune de la au moins une valeur de capteur et de la au moins une autre valeur de capteur étant adaptée pour déterminer le paramètre d'état des cheveux, et
le paramètre d'état des cheveux présentant un degré d'endommagement des cheveux sous la forme d'une teneur en acide cystéique.

2. Système selon la revendication 1,
dans lequel au moins l'un parmi le au moins un capteur et le au moins un autre capteur comprend un capteur optique pour déterminer la teneur en acide cystéique des cheveux et/ou pour déterminer la couleur des cheveux de l'utilisateur.

3. Système selon l'une des revendications précédentes,
dans lequel l'un des au moins un capteur et au moins un autre capteur comprend un microphone pour déterminer la rugosité de surface des cheveux.

4. Système selon l'une des revendications précédentes,
le dispositif de traitement de données faisant partie d'un smartphone, d'une tablette ou d'un iPad.

5. Système selon l'une des revendications précédentes,
dans lequel le premier dispositif capteur et le dispositif de traitement de données forment un dispositif portable intégré.

6. Procédé pour déterminer l'état des cheveux à l'aide d'un système selon l'une des
revendications précédentes, comprenant :
l'acquisition (210) d'au moins une valeur de capteur sur les cheveux d'un utilisateur à l'aide du dispositif de capteur portable ;
la fourniture (220) de ladite au moins une valeur de capteur au dispositif de traitement de données ;
la determination (230), à l'aide du dispositif de traitement de données (226), si ledit dispositif de traitement de données reçoit en outre au moins une autre valeur de capteur provenant dudit autre dispositif de capteur (101) ;
la determination (240a,b) d'un état capillaire de l'utilisateur à l'aide du dispositif de traitement de données sur la base de la au moins une valeur de capteur détectée dans le cas où il a été déterminé que seule la au moins une valeur de capteur est fournie, ou sur la base de la au moins une valeur de capteur fournie et de la au moins une autre valeur de capteur fournie dans le cas où il a été déterminé que la au moins une autre valeur de capteur est également fournie,
l'état des cheveux présentant un paramètre d'état des cheveux et chacune de la au moins une valeur de capteur et de la au moins une autre valeur de capteur étant appropriée pour déterminer le paramètre d'état des cheveux, et
le paramètre d'état des cheveux présentant un degré d'endommagement des cheveux sous la forme d'une teneur en acide cystéique.

7. Procédé selon la revendication 6,
la détermination de l'état des cheveux comprenant la détermination de l'humidité des cheveux de l'utilisateur.

8. Procédé pour déterminer une recommandation concernant les cheveux d'un
utilisateur, comprenant :
la détermination d'un état des cheveux selon l'une des revendications 6 ou 7 ; et
la détermination d'une recommandation concernant les cheveux de l'utilisateur sur la base de l'état des cheveux déterminé.

9. Procédé selon la revendication 8,
dans lequel la recommandation concernant les cheveux d'un utilisateur comprend au moins une recommandation de produit de traitement capillaire et une recommandation de méthode de traitement capillaire.

10. Procédé selon la revendication 8 ou 9,
permettant à l'utilisateur de commander en ligne le produit de traitement capillaire recommandé et/ou indiquant à l'utilisateur où le produit de traitement capillaire recommandé est disponible.
